(19) Europäisches Patentamt
European Patent Office
Office européen des brevets

(11) Veröffentlichungsnummer: **0 056 265**
**B1**

# (12) EUROPÄISCHE PATENTSCHRIFT

(45) Veröffentlichungstag der Patentschrift: **15.05.85**

(51) Int. Cl.[4]: **C 07 H 19/06**, A 61 K 31/70

(21) Anmeldenummer: **82100104.7**

(22) Anmeldetag: **08.01.82**

(54) **5'-Ester von Pyrimidinnucleosiden mit antiviraler Wirksamkeit, Verfahren zur Herstellung und daraus hergestellte Arzneimittel.**

(30) Priorität: **09.01.81 DE 3100478**

(43) Veröffentlichungstag der Anmeldung: **21.07.82 Patentblatt 82/29**

(45) Bekanntmachung des Hinweises auf die Patenterteilung: **15.05.85 Patentblatt 85/20**

(84) Benannte Vertragsstaaten: **AT CH DE FR GB IT LI SE**

(56) Entgegenhaltungen:
**EP-A- 0 013 625**
**DE-A- 2 658 672**
**DE-A- 3 010 399**
**FR-A- 1 336 866**
**FR-M- 3 783**
**FR-M- 4 435**
**GB-A- 1 026 586**
**US-A- 3 676 422**
**"Burger's Medicinal Chemistry", Fourth Edition, Teil I (M.E. Wolff) Wiley-Interscience, Seiten 562 und 612**
**Die Akte enthält technische Angaben, die nach dem Eingang der Anmeldung eingereicht wurden und die nicht in dieser Patentschrift enthalten sind.**

(73) Patentinhaber: **Dr. Thilo & Co. GmbH**
**Rudolf-Diesel-Ring 21**
**D-8029 Sauerlach (DE)**

(72) Erfinder: **Löbering, Hans-Georg, Dr. rer. nat**
**Vogelherd 10**
**D-8035 Gauting-Buchendorf (DE)**
Erfinder: **Miestereck, Helmut, Dr. med**
**Brammenstrasse 26**
**D-4600 Dortmund 13 (DE)**

(74) Vertreter: **von Füner, Alexander, Dr. et al**
**Patentanwälte v. Füner, Ebbinghaus, Finck Mariahilfplatz 2 & 3**
**D-8000 München 90 (DE)**

**EP 0 056 265 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

IDU (5-Jod-2'-desoxy-uridin) (P. K. Chang et al., Med. Chem. *6*, 428/1963) und Ara-T (β-D-Arabinofuranosylthymin) (G. A. Gentry, J. F. Answell, Virology *65* (1), 294/1975) sind zwei bekannte, antiviral wirksame Substanzen. Mangelnde Lipophilie hat jedoch eine schlechte Resorption beider Substanzen zur Folge. Mit Hilfe einer DMSO-Lösung von IDU hat man zwar eine verbesserte Resorption (J. Verboo, J. antimicrob. Chemother. London, *5*, 126/1979) erreichen können, die Anwendung von DMSO ist jedoch umstritten.

Aufgabe der Erfindung war daher die Synthese lipophiler Prodrugs beider Substanzen, wobei sowohl eine gute Resorption als auch anschließend eine schnelle Spaltung, d. h. eine leichte Freisetzung der Nucleoside aus den Prodrugs erreicht werden sollte.

In der US-A-3 676 422 werden zwar Verbindungen beschrieben, die durch Veresterung in ihrem Verhalten lipophiler werden, diese Verbindungen stellen jedoch keine Prodrugs dar. Sie müssen vielmehr als ein neues Virustatikum betrachtet werden. Adamantanverbindungen sind selbst virustatisch aktiv. Die esteratische Spaltung durch Enzyme ist somit nicht erforderlich, um den Wirkstoff erst in-vivo freizusetzen. Die erfindungsgemäßen Verbindungen hingegen sind in ihrer veresterten Form unwirksam, d. h. sie stellen echte Prodrugs dar, aus denen erst in-vivo das wirksame Agens freigesetzt werden muß.

Es gibt auch die ältere DE-A-3 010 399 mit einer völlig anderen Problemlösung. Im vorliegenden Fall war die Fragestellung, ein zur lokalen Anwendung ungeeignetes Virustatikum chemisch so zu verändern, daß es für die lokale Anwendung am Auge geeignet wird. Dies war nur zu erreichen durch Veresterung mit ganz bestimmten Fettsäuren. Voraussetzung für die Eignung als lokales Arzneimittel am Auge ist einmal die gute Resorbierbarkeit des Arzneimittels durch die Hornhaut, im Falle von Prodrugs die schnelle Spaltung der Transportform des Arzneimittels zum wirksamen Agens sowie eine ausreichende Wasserlöslichkeit. So ist z. B. das der erfindungsgemäßen Verbindung zugrundeliegende Virustatikum Ara-T in Wasser schwer löslich, andererseits erst in Konzentrationen von 3 % und höher am Auge virustatisch wirksam. Durch die Veresterung mit ganz bestimmten Fettsäuren läßt sich daraus ein Prodrug synthetisieren, das sowohl über eine höhere Wasserlöslichkeit verfügt als auch eine bessere Penetrationsfähigkeit durch die Hornhaut bewirkt und somit in Konzentrationen von etwa 1 % eine ausreichende, virustatische Wirksamkeit aufweist. Diese Eigenschaften sind nicht generell dadurch zu erreichen, daß das Nucleosid mit Fettsäuren verestert wird, sondern nur durch Einsatz ganz bestimmter Säuregruppierungen. Die Eigenschaften der erfindungsgemäßen Verbindungen sind somit nicht vorhersehbar gewesen und waren unerwartet.

Die in « Burger's Medicinal Chemistry », Fourth Edition, Teil I (M.E. Wolff) ; Seiten 562 + 612 zitierten 5'-O-acyl-β-D-arabinofuranosylcytosin Derivate sind aufgrund ganz anderer Überlegungen synthetisiert worden. Es ist bekannt, daß 4-Amino-Nucleoside leicht enzymatisch desaminiert werden und dabei ihre virustatische Wirksamkeit verlieren. Um diese Desaminierung zu verhindern, wurde bei den bekannten Verbindungen eine Veresterung in 5'-Stellung durchgeführt. Diese Veresterung sollte somit bewirken, daß der Angriff der Desaminase blockiert wird. Die dabei synthetisierten Ester müssen im Gegensatz zu den erfindungsgemäßen Verbindungen eine sehr hohe Stabilität aufweisen, um den erwünschten Effekt zu erzielen. Bei den zitierten Verbindungen wurde somit nicht versucht, durch Veresterung in 5'-Stellung eine Transportform des Arzneimittels zu finden, die eine bessere Bioverfügbarkeit bewirkt, sondern das Arzneimittelmolekül wurde so abgewandelt, daß bestimmte enzymatische Angriffe nicht stattfinden. Die erfindungsgemäßen Verbindungen hingegen sind bewußt so synthetisiert worden, daß ein enzymatischer Angriff, nämlich durch Esterasen, beim Durchtritt durch die Hornhaut erfolgt und das wirksame Agens freigesetzt wird. Ein Zusammenhang mit der Aufgabenstellung der Erfindung ist somit nicht gegeben.

Bekannt sind ferner 3', 5'-Diester-Derivate von IDU. Diese Diester stellen jedoch keine Prodrug-Form des ursprünglichen Arzneimittels mehr dar. Wie nachgewiesen wurde, wird der Ester in 3'-Stellung nur sehr langsam esteratisch gespalten. Bei derartigen Diestern findet somit die esteratische Spaltung nur in 5'-Stellung statt, d. h. es wird bei Diestern nicht mehr das ursprüngliche Arzneimittel freigesetzt durch enzymatischen Angriff, sondern der nicht mehr weiter spaltbare 3'-Ester, was bedeutet, daß somit eine neue Verbindung vorliegt und nicht mehr das ursprüngliche Arzneimittel. Diese unterschiedliche Spaltungsgeschwindigkeit der beiden Ester ist aufgrund der sterischen Gegebenheiten durchaus pausibel.

Die erfindungsgemäßen Verbindungen zeigen durchaus unerwartete Wirkungen und Eigenschaften gegenüber den Eigenschaften, die zu erwarten sind, wenn ein Nucleosid in 5'-Stellung verestert wird.

In diesem Zusammenhang darf nocheinmal darauf hingewiesen werden, daß bisher der durch die vorliegende Erfindung eindeutig bewiesene Zusammenhang zwischen Wirksamkeit des zugrundeliegenden Arzneimittels und Spaltung des in Form seines Esters vorliegenden Prodrugs bei Pyrimidinnucleosiden bisher noch nicht bekannt war.

Die erfindungsgemäße Aufgabe wird gelöst durch die Bereitstellung von 5'-Ester von Pyrimidinnucleosiden der allgemeinen Formel I

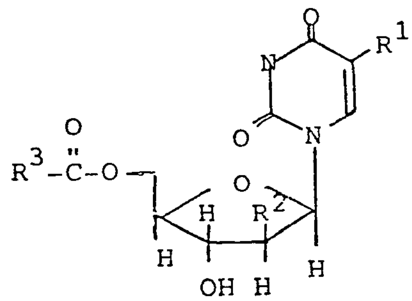

worin $R^1$ eine Methylgruppe oder ein Jodatom, $R^2$ ein Wasserstoffatom oder eine Hydroxylgruppe, $R^3$ eine verzweigte oder unverzweigte Alkylgruppe von 1 bis 17 C-Atomen, eine Cycloalkylgruppe mit 5 bis 10 C-Atomen oder eine Phenylgruppe bedeuten.

Eine bevorzugte Gruppe der erfindungsgemäßen Verbindungen umfaßt den Fall, wenn $R^2$ Wasserstoff im Falle von $R^1$ = J oder eine Hydroxylgruppe im Falle von $R^1$ = Methyl ist.

Besonders bevorzugt sind 5'-Acetyl-β-D-arabinofuranosyl-thymin, 5'-Valeroyl-β-D-arabinofuranosyl-thymin, 5-Jod-5'-valeroyl-2'-desoxy-β-D-uridin, 5'-Cyclohexanoyl-5-jod-2'-desoxy-β-D-uridin und 5'-Palmitoyl-β-D-arabinofuranosyl-thymin, 5'-Pivaloyl-β-D-arabinofuranosylthymin, 5'-Butyryl-5-jod-2'-desoxy-β-D-uridin, 5'-Benzoyl-5-jod-2'-desoxy-β-D-uridin.

Die erfindungsgemäßen Verbindungen werden erfindungsgemäß dadurch hergestellt, daß man ein Nucleosid der allgemeinen Formel II

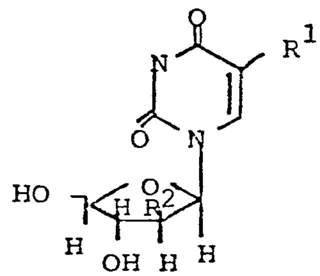

worin $R^1$ und $R^2$ die obengenannten Bedeutungen besitzen, mit Carbonsäurederivaten der allgemeinen Formel III

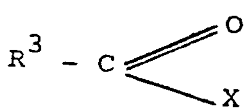

(III)

worin $R^3$ die obengenannten Bedeutungen besitzt, und X eine zur Esterbildung mit der Hydroxylgruppe in 5'-Stellung befähigte Gruppe, insbesondere eine Halogengruppe darstellt.

Vorzugsweise setzt man die Reaktionspartner in einem organischen Lösungsmittel, insbesondere Dimethylformamid oder Acetonitril unter Zugabe einer tert. Base um oder man verwendet die tert. Base, insbesondere Pyridin oder Triäthylamin gleich als Lösungsmittel.

Die Reaktionstemperatur kann vorteilhaft zwischen − 10 und + 100 °C, bevorzugt bei − 5 und + 20 °C gehalten werden.

Die erfindungsgemäßen Verbindungen können in der Pharmazie zur Herstellung von Arzneimitteln mit antiviraler Wirksamkeit eingesetzt werden.

Virustatika zur lokalen Behandlung von Viruserkrankungen am Auge und der Haut sind wie gesagt seit ca. 5 Jahren bekannt. Diesen Arzneimitteln liegen Nucleoside zugrunde, in erster Linie Ara-T, IDU, TFT, ÄDU. Allen genannten Virustatika gemeinsam ist die Eigenschaft, daß sie schlecht resorbierbar sind und deshalb hoch dosiert werden müssen, um einen therapeutischen Level bei lokaler Applikation zu erreichen. Sie sind somit für die lokale Anwendung an der Haut aufgrund der schlechten Bioverfügbarkeit in der Regel sogar ungeeignet. In einzelnen Zubereitungen wird die schlechte Resorption des Wirkstoffes, z. B. bei IDU, durch Einsatz in DMSO-Lösung verbessert. DMSO hat bekanntlicherweise aber eine Reihe von toxischen Nebenwirkungen, die in der Humanmedizin unerwünscht sind. Es wurde versucht, durch Veresterung von Haloalkylnucleosiden deren Penetrationsfähigkeit bei topischer Applikation zu verbessern. Aufgabe der Erfindung war, sowohl die Penetrationsfähigkeit bestimmter anderer Nucleoside zu verbessern als auch die Geschwindigkeit der esteratischen Spaltung zu steuern, um so eine targetorientierte Anwendung zu erreichen. So sollte z. B. eine schnelle Spaltung des Esters eine hohe, lokale Konzentration an wirksamen Virustatikum bewirken, eine langsam spaltende Prodrug

hingegen erst eine Anreicherung des Arzneistoffes an bestimmten, enzymreichen Stellen des Körpers (z. B. Leber) bewirken. Überraschenderweise weisen die erfindungsgemäßen Verbindungen eine Reihe von spezifischen Eigenschaften auf, die für die Anwendung als Virustatikum ganz bedeutende, therapeutische Vorteile bringen :

Die Lipophilie dieser Ester ist erhöht gegenüber dem zugrundeliegenden Arzneistoff, womit eine Verbesserung der Penetrationsfähigkeit in die Haut bzw. Schleimhaut erreicht wird. Trotz dieser erhöhten Lipophilie kann die Veresterung auch eine nachfolgende, bessere Wasserlöslichkeit zur Folge haben wie z. B. bei Ara-T, das ein hochwirksames, aber schlecht wasserlösliches Virustatikum darstellt und erst durch Veresterung in der 5'-Position mit Essigsäure gut wasserlöslich wird. Dadurch ist man in der Lage, wässrige Lösungen mit ausreichender Wirkstoffkonzentration herzustellen. Diese unerwarteten Eigenschaften wurden durch Bestimmung der Verteilung der erfindungsgemäßen Verbindungen in Wasser und Chloroform sowie durch Bestimmung der Penetrationsfähigkeit mittels eines in-vitro-Modells definiert.

Besonders gute Resultate erhält man durch spezifische Veresterung der 5'-Stellung des Nucleosidzuckerrestes zu lipophilen 5'-Carbonsäureestern, wobei Untersuchungen der esteratischen Spaltung mit verschiedenen Esterasetypen und mit Humanserum ergaben, daß sich unverzweigte Ester bezüglich der Spaltungsgeschwindigkeit besonders vielversprechend verhalten (Tab. 1).

Das 5'-Acetat des Ara-T besitzt die erstaunliche Eigenschaft, in Wasser wesentlich leichter löslich zu sein als das reine Ara-T. Dadurch ergibt sich die Möglichkeit, wesentlich höherprozentige wässerige Arzneimittellösungen herzustellen.

Tabelle 1

Relative Spaltungsgeschwindigkeiten durch verschiedene Esterasen zu den freien Nucleosiden.

| | 5'-Acetat | 5'-Pivalat | 5'-Butyrat | 5'-Valerat |
|---|---|---|---|---|
| Cornea + Kammerwasser von Ochsenaugen | 20 | 1 | 100 | 100 |
| Humanserum | 20 | 10 | 100 | 100 |
| Schweineleberesterase | 1 | 10 | 100 | 100 |
| Acetylcholinesterase | 100 | 1 | 10 | 10 |

100 = sehr schnell
10 = langsam
1 = sehr langsam

Die Erfindung wird anhand der folgenden Beispiele erläutert, die jedoch keinerlei Beschränkung darstellen.

Beispiel 1

5'-Acetyl-β-D-arabinofuranosyl-thymin

1 g β-D-Arabinofuranosylthymin wird in 50 ml getrocknetem Pyridin gelöst und bei – 10 °C innerhalb von 1 Stunde mit 0,32 g Acetylchlorid in 10 ml wasserfreiem Acetonitril versetzt. Nach mehrtägigem stehen im Kühlschrank wird im Vakuum eingedampft und der Rückstand zwischen Essigester und etwas Wasser verteilt. Die wässerige Phase wird noch mehrmals mit Essigester extrahiert. Die vereinigten organischen Phasen werden nach Trocknen über Natriumsulfat eingedampft und der Feststoff aus Äthanol/Pentan kristallisiert.

Die farblosen Kristalle des 5'-Acetats (0,47 g) vom Schmelzpunkt 196 °C werden in einer Reinheit von 97 % und einer Ausbeute von 40 % erhalten.

In analoger Weise erhält man die Verbindungen 2, 3, 15 und 16 (Tabelle 2 und 3).

Beispiel 2

5'-Valeroyl-β-D-arabinofuranosyl-thymin

1 g β-D-Arabinofuranosylthymin wird in 20 ml Pyridin gelöst und unter Eiskühlung bei etwa + 5 °C 0,56 g Valeriansäurechlorid in 5 ml wasserfreiem Acetonitril zugetropft. Nach mehrtägigem Stehenlassen unter Ausschluß von Feuchtigkeit werden die flüchtigen Bestandteile im Vakuum abgezogen und der ölige Rückstand zwischen Wasser und Essigester verteilt. Die Essigesterphase wird abgetrennt, mit gesättigter Natriumbicarbonatlösdung und 0,1 n HCl gewaschen, über Natriumsulfat getrocknet und eingedampft. Nach Kristallisation aus Äthanol/Heptan und aus 25 %-igem wässerigen Äthanol werden

farblose Blättchen des reinen 5'-Valeroyl-β-D-arabinofuranosyl-thymins in einer Ausbeute von 75 % (0,99 g) erhalten. Schmelzpunkt 198 °C.

Analog zu diesem Beispiel erhält man die Verbindungen 3 bis 13 und 17 bis 27.

## Beispiel 3

5-Jod-5'-valeroyl-2'-desoxy-β-D-uridin

Zu einer Suspension von 1 g Joddesoxyuridin in 28 ml Dimethylformamid werden 2 ml Pyridin gegeben und bei + 5 °C unter gutem Rühren 0,37 g Valeroylchlorid zugetropft. Nach mehrtätigem Stehen unter Feuchtigkeitsausschluß wird gemäß Beispiel 2 aufgearbeitet und man erhält nach Kristallisation aus Äthanol/Pentan farblose Blättchen des 5'-Esters in einer Ausbeute von 45 % (0,56 g). Schmelzpunkt 146 °C.

## Beispiel 4

5'-Cyclohexanoyl-5-jod-2'-desoxy-β-D-uridin

Zu 0,354 g Joddesoxyuridin in 10 ml Pyridin werden unter Eiskühlung 0,161 g Cyclohexansäurechlorid in 5 ml Acetonitril zugegeben, 2 Tage unter Feuchtigkeitsausschluß stehengelassen, kurz auf 80 °C erwärmt, die flüchtigen Bestandteile dann abgezogen und das zurückbleibende Öl zwischen Essigester und Wasser verteilt. Nach Abtrennen und Abdampfen des Essigesters wird der Rückstand mit heißem n-Heptan verührt, bis ein chromatographisch reiner Feststoff des 5'-Cyclohexanolesters vom Schmelzpunkt 105 bis 110 °C entstand. Die Ausbeute betrug 34 % (0,16 g).

Analog zu diesem Beispiel erhält man die Verbindungen 8, 10 und 22 bis 24.

## Beispiel 5

5'-Palmitoyl-β-D-arabinofuranosylthymin.

1 g β-D-Arabinofuranosylthymin wird in 20 ml Pyridin gelöst und unter Eiskühlung 1,2 g Palmitinsäurechlorid gelöst und unter Eiskühlung 1,2 g Palmitinsäurechlorid zugetropft. Dann wird auf Raumtemperatur erwärmt, zwei Tage unter Feuchtigkeitsausschluß stehengelassen und die klare Lösung im Vakuum eingedampft. Der zähe Rückstand wird mit Wasser verrührt bis zur Kristallisation, der Feststoff abfiltriert, mit Wasser und 0,1 n HCl nachgewaschen und aus Äthanol/Heptan kristallisiert.

Der farblose Feststoff des 5'-Esters vom Schmelzpunkt 179 °C entstand in einer Ausbeute von 88 % (1,7 g).

Analog diesem Beispiel erhält man Verbindungen 12, 14 und 26 bis 28.

Tabelle 2

| 5'-Acyl-ß-D-arabino-furanosylthymin 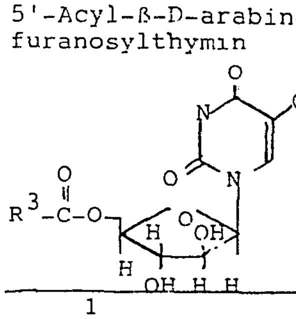 | Rest $R^3$ | Elementaranalyse | | | |
|---|---|---|---|---|---|
| | | | C % | H % | N % |
| 1 | 2 | | 3 | 4 | 5 |
| 1. 5'-Acetyl Fp 196 °C | $CH_3$ - | Ber. | 47,98 | 5,37 | 9,33 |
| | | gef. | 47,90 | 5,40 | 9,28 |
| 2. 5'-Propionyl | $C_2H_5$- | ber. | 47,39 | 5,51 | 8,50 |
| | | gef. | 47,28 | 5,57 | 8,52 |
| 3. 5'-Butyryl | $C_3H_7$- | ber. | 48,96 | 5,87 | 8,16 |
| | | gef. | 48,79 | 5,81 | 8,00 |
| 4. 5'-Valeroyl Fp 198 °C | $C_4H_9$- | ber. | 50,40 | 6,20 | 7,84 |
| | | gef. | 50,51 | 6,10 | 7,77 |

Tabelle 2 (Fortsetzung)

| 1 | 2 | 3 | 4 | 5 |
|---|---|---|---|---|
| 5. 5'-Isobutyryl | $C_3H_7-$ | ber. 48,96<br>gef. 47,33 | 5,87<br>5,56 | 8,16<br>8,17 |
| 6. 5'-Pivaloyl<br>Fp 220°C | $C_4H_9$ | ber. 50,40<br>gef. 52,00 | 6,20<br>6,11 | 7,84<br>7,67 |
| 7. 5'-Hexanoyl | $C_5H_{11}$ | ber. 53,93<br>gef. 52,99 | 6,79<br>6,77 | 7,86<br>7,88 |
| 8. 5'-Cyclopentanoyl | $C_5H_9-$ | ber. 52,01<br>gef. 51,22 | 6,00<br>5,88 | 7,58<br>7,47 |
| 9. 5'-Cyclohexanoyl | $C_6H_{11}-$ | ber. 53,24<br>gef. 51,12 | 6,31<br>6,58 | 7,30<br>7,68 |
| 10. 5'-Adamantanoyl | $C_{10}H_{16}-$ | ber. 56,08<br>gef. 54,29 | 6,50<br>6,67 | 6,23<br>6,42 |
| 11. 5'-Benzoyl | $C_6H_5-$ | ber. 54,09<br>gef. 54,11 | 4,81<br>4,85 | 7,42<br>7,43 |
| 12. 5'-Lauroyl | $C_{11}H_{23}-$ | ber. 59,98<br>gef. 59,99 | 8,24<br>8,30 | 6,36<br>6,27 |
| 13. 5'-Palmitoyl<br>Fp 179°C | $C_{15}H_{31}-$ | ber. 61,02<br>gef. 59,25 | 8,67<br>8,15 | 5,47<br>5,44 |
| 14. 5'-Stearoyl | $C_{17}H_{35}-$ | ber. 64,10<br>gef. 64,22 | 9,22<br>9,28 | 5,34<br>5,44 |

Tabelle 3

| 5'-Acyl-5-jod-2'-desoxy-ß-D-uridin | Rest $R^3$ | Elementaranalyse | | |
|---|---|---|---|---|
| | | C % | H % | N % |
| 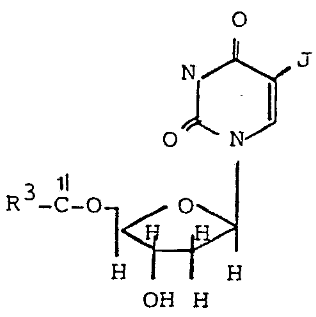 | | | | |
| 1 | 2 | 3 | 4 | 5 |
| 15. 5'-Acetyl | $CH_3-$ | ber. 33,35<br>gef. 33,44 | 3,31<br>3,52 | 7,07<br>7,18 |
| 16. 5'-Propionyl | $C_2H_5-$ | ber. 35,14<br>gef. 35,88 | 3,96<br>3,81 | 6,83<br>6,59 |
| 17. 5'-Butyryl<br>Fp 152°C | $C_3H_7-$ | ber. 36,81<br>gef. 37,25 | 4,04<br>3,96 | 6,60<br>6,72 |
| 18. 5'-Valeroyl<br>Fp 146°C | $C_4H_9-$ | ber. 38,37<br>gef. 38,36 | 4,37<br>4,29 | 6,37<br>6,29 |
| 19. 5'-Isobutyryl | $C_3H_7-$ | ber. 36,81<br>gef. 36,65 | 4,04<br>4,10 | 6,60<br>6,58 |
| 20 5'-Pivaloyl | $C_4H_9-$ | ber. 38,37<br>gef. 38,00 | 4,37<br>4,22 | 6,39<br>6,52 |
| 21. 5'-Hexanoyl | $C_5H_{11}$ | ber. 39,84<br>gef. 39,55 | 4,68<br>4,66 | 6,19<br>6,29 |

Tabelle 3 (Fortsetzung)

| 1 | 2 | 3 | 4 | 5 |
|---|---|---|---|---|
| 22. 5'-Cyclopentanoyl | $C_5H_9-$ | ber. 40,02<br>gef. 41,00 | 4,25<br>4,18 | 6,22<br>6,31 |
| 23. 5'-Cyclohexanoyl<br>Fp 105-110°C | $C_6H_{11}-$ | ber. 41,39<br>gef. 41,38 | 4,56<br>4,29 | 6,03<br>6,12 |
| 24. 5'-Adamantanoyl | $C_{10}H_{16}-$ | ber. 46,43<br>gef. 45,98 | 5,07<br>4,99 | 5,41<br>5,55 |
| 25. 5'-Benzoyl<br>Fp 168°C | $C_6H_5-$ | ber. 41,04<br>gef. 40,59 | 3,23<br>3,18 | 5,98<br>5,99 |
| 26. 5'-Lauroyl | $C_{11}H_{23}-$ | ber. 46,98<br>gef. 47,33 | 6,20<br>6,22 | 5,22<br>5,14 |
| 27. 5'-Palmitoyl | $C_{15}H_{31}-$ | ber. 50,68<br>gef. 48,71 | 6,98<br>6,65 | 4,73<br>4,73 |
| 28. 5'-Stearoyl | $C_{17}H_{35}-$ | ber. 52,26<br>gef. 53,14 | 7,31<br>7,00 | 4,51<br>4,33 |

**Patentansprüche**

1. 5'-Ester von Pyrimidinnucleosiden der allgemeinen Formel I

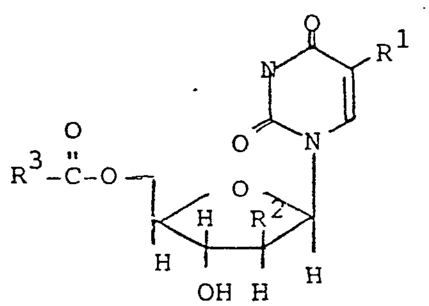

I

worin

$R^1$ eine Methylgruppe oder ein Jodatom,

$R^2$ ein Wasserstoffatom oder eine Hydroxylgruppe,

$R^3$ eine verzweigte oder unverzweigte Alkylgruppe von 1 bis 17 C-Atomen, eine Cycloalkylgruppe mit 5 bis 10 C-Atomen oder eine Phenylgruppe bedeuten.

2. Ester nach Anspruch 1, dadurch gekennzeichnet, daß $R^2$ Wasserstoff ist im Falle von $R^1$ = J oder eine Hydroxylgruppe im Falle von $R^1$ = Methyl.

3. 5'-Acetyl-β-D-arabinofuranosyl-thymin.

4. 5'-Valeroyl-β-D-arabinofuranosyl-thymin.

5. 5-Jod-5'-valeroyl-2'-desoxy-β-D-uridin.

6. 5'-Cyclohexanoyl-5-jod-2'-desoxy-β-D-uridin.

7. 5'-Palmitoyl-β-D-arabinofuranosylthymin.

8. 5'-Pivaloyl-β-D-arabinofuranosylthymin.

9. 5'-Butyryl-5-jod-2'-desoxy-β-D-uridin.

10. 5'-Benzoyl-5-jod-2'-desoxy-β-D-uridin.

11. Verfahren zur Herstellung von Verbindungen nach Anspruch 1 bis 10, dadurch gekennzeichnet, daß man ein Nucleosid der allgemeinen Formel II

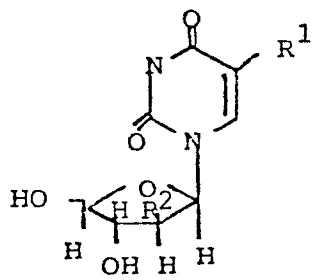

II

worin $R^1$ und $R^2$ die in Anspruch 1 genannten Bedeutungen besitzen, mit Carbonsäurederivaten der allgemeinen Formel III

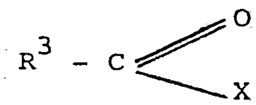

III

umsetzt worin $R^3$ die in Anspruch 1 genannten Bedeutungen besitzt und X eine zur Esterbildung mit der Hydroxylgruppe in 5'-Stellung befähigte Gruppe, insbesondere eine Halogengruppe darstellt.

12. Verfahren nach Anspruch 11, dadurch gekennzeichnet, daß man die Reaktionspartner in einem organischen Lösungsmittel, insbesondere Dimethylformamid oder Acetonitril unter Zugabe einer tert. Base umsetzt oder die tert. Base, insbesondere Pyridin oder Triäthylamin, gleich als Lösungsmittel verwendet.

13. Verfahren nach einem der Ansprüche 11 oder 12, dadurch gekennzeichnet, daß die Reaktionstemperatur zwischen − 10 und + 100 °C, bevorzugt bei − 5 bis + 20 °C, gehalten werden kann.

14. Arzneimittel enthaltend Verbindungen nach den Ansprüchen 1 bis 10.

**Claims**

1. 5'-esters of pyrimidinenucleosides of the general formula I

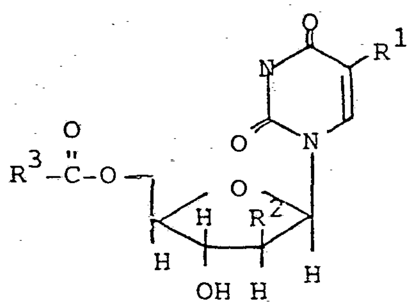

in which

$R^1$ represents a methyl group or an iodine atom,

$R^2$ represents a hydrogen atom or a hydroxyl group,

$R^3$ represents a branched or unbranched alkyl group of 1 to 17 C atoms, a cycloalkyl group with 5 to 10 C atoms, or a phenyl group.

2. Esters according to claim 1, characterised in that $R^2$ is hydrogen when $R^1$ = I, or a hydroxyl group when $R^1$ = methyl.

3. 5'-acetyl-β-D-arabinofuranosyl-thymine.

4. 5'-valeroyl-β-D-arabinofuranosyl-thymine.

5. 5-iodo-5'-valeroyl-2'-desoxy-β-D-uridine.

6. 5'-cyclohexanoyl-5-iodo-2'-desoxy-β-D-uridine.

7. 5'-palmitoyl-β-D-arabinofuranosylthymine.

8. 5'-pivaloyl-β-D-arabinofuranosylthymine.

9. 5'-butyryl-5-iodo-2'-desoxy-β-D-uridine.

10. 5'-benzoyl-5-iodo-2'-desoxy-β-D-uridine.

11. Process for the manufacture of compounds according to claims 1 to 10, characterised in that a nucleoside of the general formula II

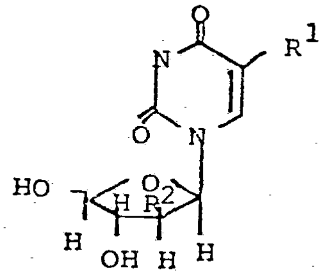

II

in which $R^1$ and $R^2$ have the meanings given in claim 1 is reacted with carboxylic acid derivatives of the general formula III

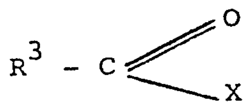

III

in which $R^3$ has the meanings given in claim 1, and X represents a group, especially a halogen group, capable of esterification with the hydroxyl group in the 5′ position.

12. Process according to claim 11, characterised in that the reactants are reacted in an organic solvent, especially dimethylformamide or acetonitrile, with the addition of a tertiary base, or the tertiary base, especially pyridine or triethylamine, is used directly as the solvent.

13. Process according to one of claims 11 or 12, characterised in that the reaction temperature can be held between − 10 and + 100 °C, preferably at − 5 to + 20 °C.

14. Medicinal compositions containing compounds according to claims 1 to 10.

**Revendications**

1. Esters 5′ de pyrimidine-nucléosides de formule générale I

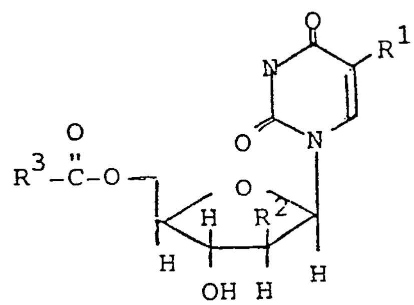

I

où

$R^1$ représente un groupe méthyle ou un atome d'iode ;

$R^2$ représente un atome d'hydrogène ou un groupe hydroxyle ;

$R^3$ représente un groupe alkyle ramifié ou non ramifié renfermant de 1 à 17 atomes de carbone, un groupe cycloalkyle renfermant 5 à 10 atomes de carbone ou un groupe phényl.

2. Ester selon la revendication 1, caractérisé en ce que $R^2$ est de l'hydrogène dans le cas où $R^1$ = I ou un groupe hydroxyle dans le cas où $R^1$ = méthyle.

3. 5′-acétyl-β-D-arabinofuranosyl-thymine.

4. 5′-valéroyl-β-D-arabinofuranosyl-thymine.

5. 5-iode-5′-valéroyl-2′-désoxy-β-D-uridine.

6. 5′-cyclohexanoyl-5-iode-2′-désoxy-β-D-uridine.

7. 5′-palmitoyl-β-D-arabinofuranosyl-thymine.

8. 5′-pivaloyl-β-D-arabinofuranosyl-thymine.

9. 5′-butyryl-5-iode-2′-désoxy-β-D-uridine.

10. 5′-benzoyl-5-iode-2′-désoxy-β-D-uridine.

11. Procédé pour la préparation de composés selon les revendications 1 à 10, caractérisé en ce que l'on fait réagir un nucléoside de la formule générale II

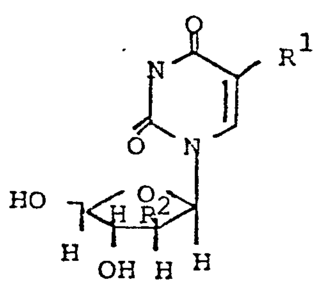

II

où $R^1$ et $R^2$ ont les significations indiquées dans la revendication 1, avec des dérivés d'acides carboxyliques de formule générale III

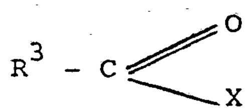

III

où $R^3$ a les significations indiquées dans la revendication 1, et X représente un groupe capable de former un ester avec le groupe hydroxyle en position 5', notamment un halogène.

12. Procédé selon la revendication 11, caractérisé en ce que l'on fait réagir les réactifs dans un solvant organique, notamment le diméthylformamide ou l'acétonitrile en ajoutant une base tertiaire, ou que l'on utilise la base tertiaire, notamment la pyridine ou la triéthylamine elle-même en tant que solvant.

13. Procédé selon une des revendications 11 ou 12, caractérisé en ce que l'on peut maintenir la température de réaction entre − 10 et + 100 °C, de préférence entre − 5 à + 20 °C.

14. Médicament renfermant des composés selon les revendications 1 à 10.